## Europäisches Patentamt

## European Patent Office

## Office européen des brevets

(11) Numéro de publication: **0 217 845 B1**

(12) **FASCICULE DE BREVET EUROPEEN**

(45) Date de publication de fascicule du brevet: **19.01.94**

(21) Numéro de dépôt: **86901901.8**

(22) Date de dépôt: **19.03.86**

(86) Numéro de dépôt internationale :
**PCT/FR86/00094**

(87) Numéro de publication internationale :
**WO 86/05591 (25.09.86 86/21)**

Demande divisionnaire 90200840.8 déposée le
19/03/86.

(51) Int. Cl.⁵: **G01N 33/543**, G01N 33/80,
G01N 33/569

(54) PROCEDE DE DETERMINATION IMMUNOLOGIOUE D'UNE SUBSTANCE BIOLOGIOUE DANS UN ECHANTILLON.

(30) Priorité: **19.03.85 FR 8504013**

(43) Date de publication de la demande:
**15.04.87 Bulletin 87/16**

(45) Mention de la délivrance du brevet:
**19.01.94 Bulletin 94/03**

(84) Etats contractants désignés:
**AT BE CH DE FR GB IT LI LU NL SE**

(56) Documents cités:
**EP-A- 32 204      EP-A- 32 782**
**EP-A- 41 426      EP-A- 106 685**
**WO-A-84/03151      GB-A- 2 117 789**
**US-A- 3 990 850**

**PATENT ABSTRACTS OF JAPAN, vol. 9, no.
72 (P-345)(1795) 2 avril 1985&NUM;**

(73) Titulaire: **LEE, Helen Hwai-an**
**3, route des Gatines**
**F-78190 Elancourt(FR)**

Titulaire: **CANAVAGGIO, Michel Etienne**
**25 bis, rue Jasmin**
**F-75016 Paris(FR)**

(72) Inventeur: **LEE, Helen Hwai-an**
**3, route des Gatines**
**F-78190 Elancourt(FR)**
Inventeur: **CANAVAGGIO, Michel Etienne**
**25 bis, rue Jasmin**
**F-75016 Paris(FR)**

(74) Mandataire: **Phélip, Bruno et al**
**c/o Cabinet Harlé & Phélip**
**21, rue de La Rochefoucauld**
**F-75009 Paris (FR)**

Rank Xerox (UK) Business Services
(3.10/3.09/3.3.3)

## Description

La présente invention concerne un procédé d'identification , et éventuellement de dosage, de substances biologiques.

Les procédés existants pour détecter des substances biologiques sont nombreux. Parmi ces procédés, les plus courants sont l'hémagglutination, les immuno-essais fluorescents, enzymatiques ou radioactifs .

Les immunoessais mentionnés précédemment nécessitent la mise en oeuvre de produits de couplage artificiels tels que des conjugués anticorps-enzyme ou un marquage radioactif.

Deux demandes européennes , EP-A-032 204 et EP-A-032 782 ont pour objet des méthodes de détection d'enzymes à l'aide d'anticorps fixés sur une phase solide . Dans le cas de la demande EP-A-032 204, l'enzyme détecté est la phosphatase alcaline tandis que dans le cas de la demande EP-O32 782 l'enzyme détecté est l'hémoglobine. Dans les deux cas, on dose un enzyme activé à l'aide d'un anticorps fixé sur une phase solide et non pas un pro-enzyme dépourvu de toute activité enzymatique.

La demande WO-A-8403151 concerne une méthode de détection en phase liquide, c'est-à-dire en phase homogène , comprenant la réaction de cellules et d'anticorps spécifiques d'antigènes de surface de ces cellules puis leur lyse par du complément.

La détection de la réaction se fait à l'oeil nu et sans amplification, la coloration naturelle des cellules étant seule utilisée . Cette méthode est donc peu sensible.

La demande européenne EP-041.426 concerne un procédé de dosage immunologique de type sandwich utilisant comme révélateur un conjugué .La demande EP-A-106.185 décrit une méthode de détermination des groupes sanguins utilisant des conjugués fluorescents, et non les propriétés intrinsèques des cellules.

Ces procédés n'utilisent donc pas les caractéristiques inhérentes à la structure des cellules .

Le brevet US-A-3 99O.65O est relatif à une nouvelle présentation d'un test de diagnostic dans lequel les réactifs sont simplement séchés sur des dispositifs de réaction et non fixés .

La présente invention a pour objet un procédé n'ayant pas recours à des produits de couplage artificiels ou à des produits radioactifs et qui puisse être mis en oeuvre en phase solide, à la différence de l'hémagglutination. Ce procédé présente, en particulier, l'avantage d'être simple, rapide et de pouvoir être automatisé tout en étant très sensible

Plus particulièrement, la présente invention concerne un procédé de détermination d'une cellule ou d'un micro-organisme dans un échantillon, caractérisé en ce que:

a) on immobilise sur une phase solide un ligand ayant une affinité de fixation pour ladite cellule ou ledit micro-organisme ;

b) on met à incuber l'échantillon en présence de ladite phase solide sur laquelle est immobilisé le ligand ;

c) on lave la phase solide après incubation ; et

d) on révèle la présence de ladite cellule ou dudit micro-organisme, fixée sur la phase solide par l'intermédiaire du ligand, par mise en évidence d'une activité enzymatique ou chimique endogène de ladite cellule ou dudit micro-organisme après lyse.

Le procédé selon la présente invention permet de mettre en évidence toutes les cellules ou micro-organismes qui seront nommées ci-après "autorévélables", c'est-à-dire toutes les cellules ou micro-organismes qui possèdent une propriété inhérente qui peut être mise directement ou indirectement en évidence pour l'homme ou la machine.

Les substances autorévélables sont, en particulier, les substances présentant une coloration naturelle, par exemple les globules rouges, ou bien présentant une apparence particulièrement sous un rayonnement artificiel, ou encore les substances capables, directement lorsqu'il s'agit d'une macromolécule, ou grâce à certains éléments qu'elles contiennent, enzyme endogène ou composant chimique par exemple, de colorer un substrat ou de générer une fluorescence ou une luminescence, protéine C par exemple.

Bien entendu, les "substances autorévélables" peuvent être révélées par de nombreuses autres propriétés chimiques ou physiques.

De façon générale, le procédé selon la présente invention est plus particulièrement destiné à la détection de substances biologiques directement visualisables en lumière naturelle ou artificielle. Dans ce cas, il s'agit en général de substances colorées ou pigmentées et qui sont présentes en forte concentration.

Ce procédé est, en outre, particulièrement intéressant pour la détection de substances biologiques révélables indirectement par une coloration ou une réaction enzymatique endogène, en présence d'un substrat et éventuellement après activation enzymatique ou lyse cellulaire s'il s'agit de cellules, libérant des enzymes endogènes notamment peroxydase, phosphatase, galactosidase, glucose-oxydase, transaminase par exemple.

2

Les substances biologiques autorévélables peuvent être :

1°) Des cellules, en particulier :

- des globules rouges pour la détermination des groupes sanguins, des systèmes ABH, Lewis, P, Lutheran, Rhésus (D, C, c, E, e) Kell, Duffy, Kidd et MNSs ou autres, révélables par leur coloration ou après lyse par l'activité de la peroxydase sur un substrat ;
- des leucocytes : polynucléaires, lymphocytes, monocytes ;
- des plaquettes sanguines ;
- des cellules d'origine épithéliale, endothéliale ou conjonctives normales ou pathologiques présentes dans le liquide de prélèvement ou prélevées par cytoponction ou biopsie et resuspendues dans un tampon artificiel.

2°) Des microorganismes : bactéries, parasites, virus, les bactéries étant, par exemple, révélables après lyse par l'activité d'enzymes endogènes comme la galactosidase.

Le produit autorévélé peut être directement sous forme active ou être secondairement activé par lyse cellulaire et libération dans le milieu réactif ou par activation secondaire dans une cascade enzymatique.

La phase solide peut être n'importe quel support habituellement utilisé pour les immunoessais , qu'il s'agisse de support en forme de bille, de puits sur des plaques de microtitration, de bandelettes réactives ou autres . Ce support peut être en n'importe quel polymère d'origine naturelle ou synthétique ou en substance amorphe tel le verre . On utilise avantageusement pour les bandelettes réactives les membranes de nitrocellulose ou de nylon, pour les billes ou microplaques , les matières plastiques polyvinyl, polypropylène , polystyrène ou autre . On peut encore utiliser des gels ou des particules à base d'agarose, d'acrylamide ou de latex.

La sensibilisation de la phase solide par le ligand ayant une affinité vis-à-vis de la substance à doser est faite soit par adsorption passive, soit par couplage covalent selon la nature du support et des impératifs du test. La phase solide , après adsorption du ligand , peut être saturée par des protéines ou macromolécules telles que l'albumine bovine, le sérum de veau foetal, la gélatine, ou chimiquement modifiée par des agents chimiques comme le Tween®, peut empêcher les interactions non spécifiques . Cette phase solide peut être modifiée pour être conservée sous forme desséchée ou lyophilisée. Le ligand peut être une lectine, un antigène ou le plus souvent un anticorps.

Dans un mode de mise en oeuvre particulièrement intéressant de l'invention,la phase solide est constituée par une bandelette réactive ou une plaque sensibilisée par au moins deux ligands distincts, spécifiques de différentes substances biologiques susceptibles d'être présentes dans l'échantillon à tester, par exemple un ligand pour les globules rouges de groupe A et un ligand pour les globules rouges du groupe B. Dans ce cas, une mise en contact de cette bandelette ou de cette plaque avec un échantillon sang permet une détermination immédiate du groupe sanguin , la plage correspondante apparaissant colorée.

Chaque plage comportant un ligand peut comporter une indication permettant pour l'homme ou la machine d'identifier la nature du résultat, code alphanumérique ou code à barres par exemple.

Afin de faciliter l'utilisation de tels supports, il est également possible de déposer les différents ligands sur la bandelette selon un schéma propre, par exemple sous la forme d'une lettre ou d'un signe en relation avec le phénotype en cause.

Ainsi, on peut prévoir des bandelettes sur lesquelles les anticorps anti-A sont sous la forme d'un A, les anticorps anti-B sous la forme d'un B et les anticorps anti-AB sous la forme de AB. De cette façon , l'attachement des globules rouges à l'anticorps spécifique permet de lire directement le phénotype qui apparaît sous forme d'une lettre rouge.

Ce système permet un groupage sanguin en environ 5 minutes, ce qui ne peut être atteint par aucune des méthodes connues.

Les substances biologiques autorévélables sont mises à incuber en présence de la phase solide en milieu aqueux. Ce milieu peut être un liquide biologique normal ou pathologique, tel le sang, le sérum, le plasma, l'urine, le liquide cephalorachidien, un liquide d'épanchement plural, péritonéal, synovial ou autre, ou un milieu artificiel isotonique comportant éventuellement des substances d'optimisation (protéines, sels, macromolécules, agents chimiques tel le Tween®) dans lequel le prélèvement est resuspendu. L'incubation se fait à température ambiante ou modifiée avec ou sans agitation pendant un temps qui dépendra de la nature de la substance et de sa capacité à se fixer sur le ligand. Après incubation, la phase liquide est éliminée par séparation de phase ou lavage par exemple dans une solution telle un tampon PBS Tween à O,O5 %.

Le procédé selon la présente invention est plus particulièrement utilisable pour la mise en évidence de phénotypes de globules rouges correspondant à des antigènes de surface en utilisant, à titre de ligand, les anticorps monoclonaux correspondants.

La révélation de la fixation des globules rouges peut être directe grâce à la coloration rouge apparaissant, ou bien, pour rendre le procédé plus sensible, les cellules fixées après lavage sont lysées par un agent d'hémolyse et l'on dose une activité enzymatique, en particulier l'activité de la peroxydase du lysat. Le dosage de la peroxydase est connu, il peut mettre en oeuvre un substrat chromogène comme l'orthophénylène-diamine (OPD) ou le TMB.

L'intérêt d'utiliser l'activité peroxydasique de l'hémoglobine provient du fait que, bien qu'il existe de nombreuses autres enzymes dans les globules rouges, des déficits sont toujours possibles, alors qu'un déficit total ou très important en hémoglobine serait léthal.

La présente invention a en outre pour objet un coffret pour la détermination du groupe sanguin comportant:
- une phase solide selon la présente invention revêtue de différents anticorps dans des plages différentes, chacun étant spécifique d'un groupe sanguin ;
- une solution de lavage;
- un substrat chromogène; et
- un agent de lyse.

Les exemples ci-après sont destinés à illustrer d'autres caractéristiques et avantages du procédé selon la présente invention.

EXEMPLE 1 :

DETERMINATION DU GROUPE SANGUIN DANS LE SYSTEME ABH.

Des anticorps monoclonaux purifiés anti-A, anti-B, sont adsorbés passivement sur une plaque de microtitration type plaque Microelisa, fond en U rigide en immulon de référence M 124 B du Laboratoire Dynatech, ou plaque de microtitration souple, PVC, à fond en U de référence M 24 du même laboratoire. Des anticorps anti-A + B peuvent également être utilisés. Pour ce faire, les anticorps monoclonaux purifiés sont mis à incuber à la concentration de 4 $\mu$g/ml dans du tampon bicarbonate O,1 M pH 9,6 , 3 heures à 37°C, à raison de 100 $\mu$l par puits, la plaque étant recouverte d'un film adhésif. La plaque est saturée pendant 3O minutes par une solution de sérum de veau foetal à 10%.

Des échantillons de sang total prélevé sur tube EDTA, citrate ou héparine des groupes A, B, AB ou O, ainsi que des suspensions globulaires à 1 % de groupes faibles $A_2$, $A_3$, $A_x$, $A_{end}$, $B_3$ $A_1B_x$, cis AB, sont répartis à raison de 100 $\mu$l par cupule et mis à incuber à température ambiante pendant 3O minutes.

La plaque est lavée par lavage automatique Multiwash Dynatech pendant 4 cycles par du tampon PBS O,15 M pH 7,4 contenant du Tween 2O® a O,05 % . La réaction peut être spontanément lue pour les globules rouges des groupes A et AB avec les anticorps anti-A pour les globules rouges des groupes B et AB avec les anticorps anti-B.

Cette réaction positive se matérialise par une adsorption macroscopique des globules rouges, sur la surface des cupules revêtues d'anticorps, en cas de réaction négative, aucun globule n'est visible (par exemple avec les globules rouges du groupe O). Pour sensibiliser la lecture ou la rendre objective, la réaction peut être révélée après lyse des globules rouges par la réaction peroxydasique de l'hémoglobine. Pour ce faire, une solution hémolysante contenant un substrat de la peroxydase est ajoutée dans les cupules : orthophénylène-diamine (OPD) à 3 mg/ml dans du tampon citrate O,1 M à pH en présence d'$H_2O_2$ et contenant de la saponine à O,1 % comme agent d'hémolyse. Après 1O minutes, la réaction est stoppée par l'addition de 5O $\mu$l d'$H_2SO_4$ à 1,5 M. Les réactions sont lues au spectrophotomètre à 49O nm.

Dans cet exemple, les prélèvements des différents groupes normaux ou faibles (peu de sites antigéniques) donnent des réactions positives supérieures à 1,5 de densité optique, les groupes très faibles ayant très peu de sites antigéniques en particulier ($A_x$, $A_{end}$, $A_1B_x$, cis AB) donnent une densité optique comprise entre O,15 et 1,5 ou dépassant 1,5. Le bruit de fond reste dans tous les cas inférieur à O,O5O.

Le tableau I donne un exemple de résultat comparativement à la technique classique d'agglutination. Les résultats sont exprimés en DO à 49O nm pour le test selon l'invention et cotés en nombre de croix selon l'intensité comme il est classique pour l'agglutination.

4

TABLEAU I

Réactivité d'un panel de globules rouges de différents phénotypes mesurée en DO (ligne supérieure) et en agglutination cotée en croix (ligne inférieure)

| | $A_1$ | $A_2$ | $A_3$ | $A_3$ | $A_x$ | $A_x$ | $A_{end}$ | B | $B_3$ | $A_1B_x$ | CisAB | O |
|---|---|---|---|---|---|---|---|---|---|---|---|---|
| Anti-A | 1,5 | 1,5 | 1,5 • 1,5 | 1,5 | 0,184 | 0,822 | 0,647 | 0,002 | 0,005 | 1,5 | 1,5 | 0,000 |
| | +++ | +++ | +++ | +++ | - | (+) | (+) | - | - | +++ | +++ | - |
| Anti-B | 0,004 | 0,001 | 0,005 | 0,002 | 0,000 | 0,001 | 0,000 | 1,5 | 1,5 | 0,380 | 1,5 | 0,000 |
| | - | - | - | - | - | - | - | +++ | + | - | +++ | - |

Ces résultats montrent une plus grande sensibilité pour la technique de l'invention qui est capable de détecter sans ambiguïté les échantillons de groupes faibles ou particuliers comme $A_x$, $A_{end}$, $A_1 B_x$ ou cisAB, alors que ceux-ci sont faiblement dépistés ou négatifs en agglutination. Le procédé de l'invention pour la détermination des groupes ABH a été validé comparativement à la méthode d'agglutination sur 2000 prélèvements sanguins de donneurs de sang et sur des échantillons de globules rouges rares sélectionnés pour leur faible densité en site antigénique ou particularité.

EXEMPLE 2

DETERMINATION DES GROUPES SANGUINS DU SYSTEME ABH SUR MEMBRANE DE NITROCELLULOSE OU NYLON

1°) Préparation des membranes

Des bandelettes de membrane nylon Biodyne TM du Laboratoire Pall sont découpées aux dimensions désirées. Si le test est fait en tube de Kahn, les bandelettes peuvent être découpées aux dimensions intérieures du tube.

La membrane est sensibilisée par des anticorps monoclonaux anti-A et anti-B qui sont déposés sous forme liquide, soit à la pipette, soit avec un tampon imbibé de la solution d'anticorps. De manière à identifier la réaction effectuée, un tampon an forme de A pour l'anti-A et en forme de B pour l'anti-B est déposé sur la membrane. Les dépôts peuvent se faire également sous forme d'autres signes d'identification ou codes lisibles par l'opérateur ou par une machine. Si l'on utilise des anticorps purifiés, ceux-ci sont déposés à une concentration de 10 $\mu$g/ml ou éventuellement une concentration différente selon les résultats expérimentaux.Des ascites diluées ou des surnageants de culture contenant l'anticorps peuvent également être utilisés.

Les membranes sont séchées 2 heures à température ambiante ou 30 minutes à 37°C en étuve sèche. Après séchage la membrane est levée en tampon PBS à pH 7,4 puis saturée par une solution protéique, telle l'albumine bovine à 1% dans du tampon PBS ou en sérum de veau foetal à 10% ou la gélatine à 1%.

A ce stade, les membranes peuvent être employées immédiatement ou conservées après dessication pour une application ultérieure.

2°) Test de groupage proprement dit

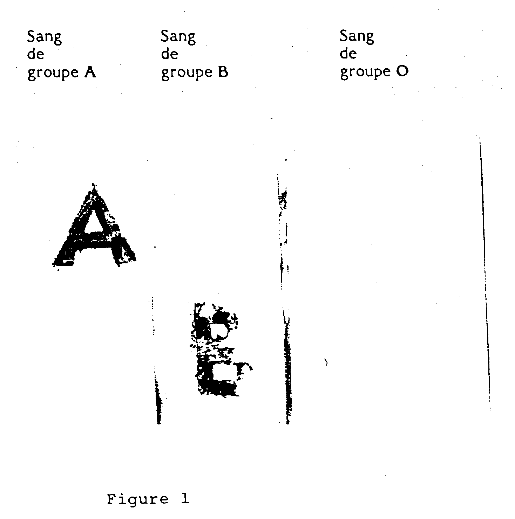

Les bandelettes sensibilisées sont immergées dans un tube contenant du sang total ou une suspension globulaire pendant 3 minutes à température ambiante avec ou sans agitation. A l'issue de l'incubation, elles sont rincées par une solution de chlorure de sodium O,15M et immédiatement lues. Si le sang est du groupe A, la lettre A apparaît, matérialisée par la fixation des globules rouges; si le sang est du groupe B, la lettre B apparaît; si le groupe est AB, les deux lettres apparaissent; si les globules sont de groupe O, la bandelette reste entièrement blanche (Figure 1).

Le procédé de l'invention est également applicable eu dépistage sans le sérum ou plasma humain des anticorps anti-érythrantigènes. Dans ce qui suit, on illustrera d'abord une première méthode de mise en oeuvre dudit procédé. Il s'agit d'un test sandwich où les anticorps à dépister sont immunoadsorbés sur le phase solide sensibilisée par les érythrentigènes et révélés par la fixation spécifique de globules rouges humains. Cette première méthode est illustrée à l'Exemple 3.

EXEMPLE 3

Détection des anticorps anti A, anti B, ou anti AB

1°) Les érythrantigènes A ou B sont adsorbés passivement sur une plaque de microtitration type plaque Microélisa, fond en U rigide en "Immulon" de référence M 124B du Laboratoire Dynatach, ou plaque de microtitration souple, PVC à fond en U de référence M 24 du même laboratoire.

Les antigènes A ou B peuvent provenir de différentes sources :
- antigènes solubles d'origine humaine présents dans le salive de certains sujets ou extraits de mucus en particulier de kystes ovariens.
- antigènes d'origine animale, tels le cheval ou le porc (extraits du mucus gastrique).
- antigènes extraits de la membrane des globules rouges humains de groupe $A_1$, $A_2B$ ou AB. Dans ce cas,des stromas de globules rouges sont préparés (par exemple selon la méthode décrite par GARDAS, A et KOSCISLAK J., dans Vox Sang., 1971, 20, 2, 137-149). Les érythrantigènes sont extraits de la membrane pendant 15 minutes à 4°C en présence d'une solution à 2% w/v d'octyl-glucoside (Laboratoire SIGMA) et d'ammoniaque à 5% v/v. D'autres agents dissociants peuvent être employés tels le Triton X 100 à 1%. La préparation est centrifugée 30 minutes à 15.OOO g pour éliminer le matériel insoluble.

Cet extrait brut d'antigène peut être employé directement après dilution en tampon phosphate O,O5 M pH 7,4 pour sensibiliser les plaques de microtitration ou faire l'objet d'étapes de purification supplémentaire.
- antigène de synthèse couplé ou non à un support macromoléculaire telle l'albumine bovine.
- des anticorps anti-idiotypes peuvent être également utilisés pour sensibiliser le phase solide.

Pour l'adsorption passive sur les plaques de microtitration, les antigènes sont dilués à une concentration de l'ordre de 10 μg/ml dans du tampon phosphate O,O5 M pH 7,4 pour les antigènes extraits des membranes erythrocytaires, ou dans un tampon bicarbonate O,1% M pH 9,6 pour des antigènes solubles.

Pour ce faire, la dilution d'antigène est mise à incuber 3 heures à 37°C à raison de 200 μl par puits, le plaque étant recouverte d'un film adhésif.

La plaque est saturée pendant 30 minutes par une solution de sérum de veau foetal à 1O% ou d'albumine bovine à 1%. Après lavage de la plaque en tampon PBS O,15 M pH 7,4 Tween 20, O,O5%, les échantillons de plasma ou sérum à étudier sont répartis à raison de 150 μl par puits, puis une solution globulaire à 2 ‰ des phénotypes A₁, A₂, B, AB ou O est ajoutée à raison de 50 μl par puits. Pour sensibiliser la réaction les globules peuvent être traitées par des enzymes telles que la papaine.

La plaque est incubée 30 minutes à température ambiante puis levée par lavage automatique pendant 4 cycles dans un tampon PBS O,15 M pH 7,4 Tween 20 O,O5%.

Les réactions peuvent être lues spontanément pour les sérums anti A, anti B ou anti AB, respectivement avec les globules rouges A₁ et A₂ pour l'anti A, B pour l'anti B, A₁, A₂ ou B pour l'anti AB.

Cette réaction positive se matérialise par une adsorption macroscopique des globules rouges, sur la surface des cupules revêtues d'anticorps, en cas de réaction négative, aucun globule n'est visible (par exemple avec les globules rouges du groupe O). Pour sensibiliser la lecture ou la rendre objective, la réaction peut être révélées après lyse des globules rouges par la réaction péroxydasique de l'hémoglobine. Pour ce faire, une solution hémolysante contenant un substrat de la péroxydase est ajoutée dans les cupules : orthophénylène-diamine (OPD) à 3 mg/ml dans du tampon citrate O,1 M à pH en présence d'$H_2O_2$ et contenant de la saponine à O,1% comme agent d'hémolyse. Après 10 minutes, la réaction est stoppée par l'addition de 50 μl d'$H_2SO_4$ à 1,5 M. Les réactions sont lues au spectrophotomètre à 490 nm.

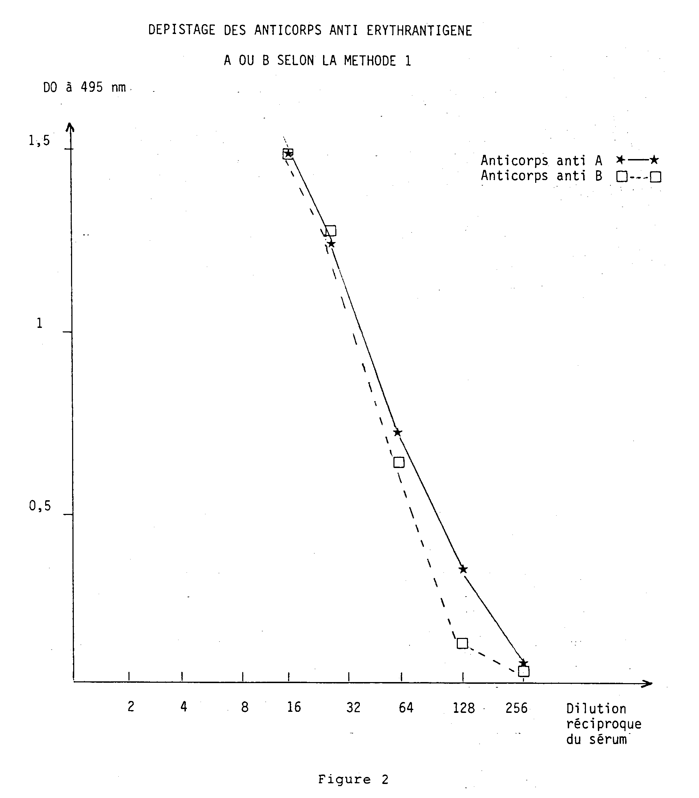

La Figure 2 est un diagramme illustrant les résultats obtenus avec la méthode (méthode I) de l'Exemple 3 dans le cas du titrage d'un sérum contenant un anticorps anti-A et d'un sérum contenant un anticorps anti-B, respectivement. On a porté en abscisses le dilution réciproque du sérum et en ordonnées le densité optique (DO) à 495 nm. Cette Figure 2 met en évidence que la sensibilité du dosage de l'invention est au moins égale à celle de la technique classique d'agglutination en tubes.

Le procédé de dépistage des anticorps anti-érythrantigènes peut également être mis en oeuvre par une deuxième méthode, qui constitue un test en deux étapes où l'échantillon contenant l'anticorps anti globules rouges à dépister est mis à incuber en présence d'hématies humaines de différents phénotypes. Après élimination des anticorps n'ayant pas réagi avec les hématies (par passage sur un filtre macromoléculaire ou lavage), les globules rouges sont mis à incuber avec une phase solide revêtue d'anti-immunoglobuline humaine de manière à ne retenir spécifiquement sur la phase solide, que les hématies ayant réagi avec les anticorps à dépister. Cette méthode peut remplacer avec plus de sensibilité et avec un résultat quantitatif, le dépistage par test de Coombs des anticorps anti globules rouges éventuellement présents dans le sérum de certains malades ou donneurs de sang et qui pourraient en l'absence de détection entraîner des accidents hémolytiques après transfusion. Cette méthode II est illustrée à l'Exemple 4.

EXEMPLE 4 : Détection d'anticorps anti Rhésus D.

Des anticorps anti-immunoglobuline humaine, purifiés par chromatographie d'affinité ou monoclonaux reconnaissant les IgG et les IgM humaines ou un mélange d'anti IgG et d'anti IgM humaine, sont adsorbés passivement sur une plaque de microtitration type plaque Microelisa à fond en V ou en U .Pour ce faire, les anticorps sont mis à incuber à une concentration de 1O μg/ml dans du tampon bicarbonate O,1 M pH 9,6 3 heures à 37°C sous un volume de 1OO μl par puits. La phase solide est lavée par lavage automatique en tampon PBS O,15 M pH 7,4 contenant du Tween 20 à O,O5%.

Parallèlement un mélange à parties égales d'une suspension à O,2 % d'hématies de phénotype Rhésus D est mis à incuber 5 à 1O minutes avec le plasma ou sérum dans lequel on recherche des anticorps anti Rhésus D. Les globules rouges ainsi incubés sont, après lavage en solution saline ou filtrage sur une solution macromoléculaire, mis en contact avec la plaque de microtitration revêtue d'anti-immunoglobuline humaine pour une incubation de 30 minutes à température ambiante.

Les plaques sont lavées par lavage automatique avec du PBS O,15 M pH 7,4 contenant du Tween 2O à O,O5%.

Les réactions peuvent être lues spontanément pour les sérums contenant des anticorps anti Rhésus D avec des hématies porteuses d'antigènes Rhésus D.

Les réactions positives se matérialisent par une adsorption macroscopique des globules rouges sur la surface des cupules revêtues d'anticorps; en cas de réaction négative, aucun globule n'est visible . Pour sensibiliser la lecture ou la rendre objective, la réaction peut être révélée après lyse des globules rouges par la réaction péroxydasique de l'hémoglobine. Pour ce faire, une solution hémolysante contenant un substrat de la péroxydase est ajoutée dans les cupules:orthophénylène-diamine (OPD) à 3 mg/ml dans du tampon citrate O,1 M à pH en présence d'$H_2O_2$ et contenant de la saponine à O,1 % comme agent d'hémolyse. Après 1O minutes, la réaction est stoppée par l'addition de 50 $\mu$l d'$H_2SO_4$ à 1,5 M. Les réactions sont lues au spectrophotomètre à 490 nm.

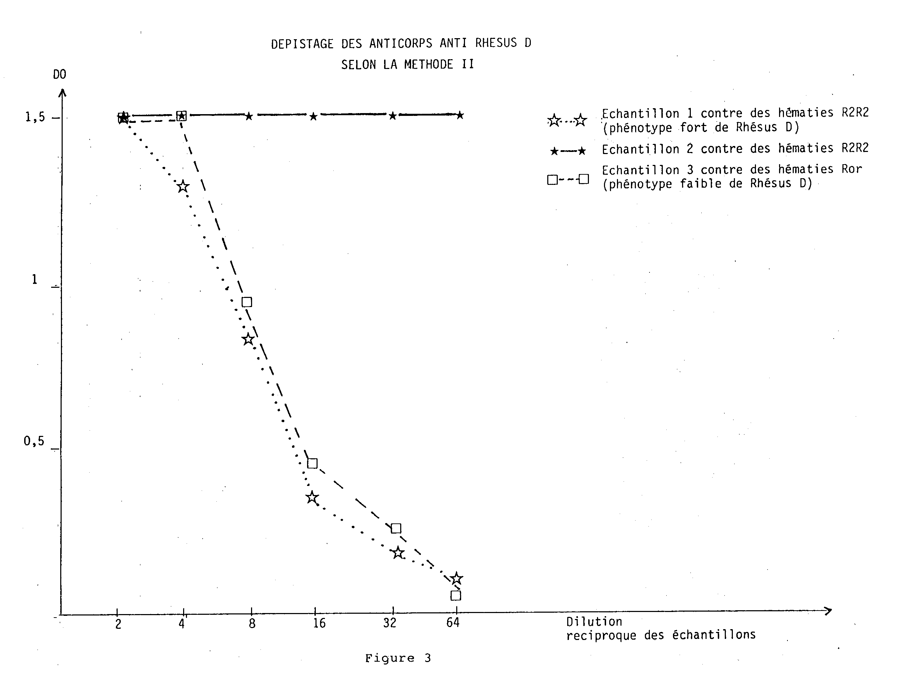

La figure 3 est un diagramme illustrant les résultats obtenus avec la méthode (méthode II) de l'exemple 4. Pour les essais rapportés à la figure 3, on a utilisé trois échantillons de sérums préalablement ramenés à une dilution correspondant à la dernière dilution positive selon la méthode de Coombs. Comme sur la figure 2, on a porté en abscisses les valeurs de la dilution et en ordonnées la densité optique DO mesurée à 495 nm. Les courbes portées sur la figure 3 montrent que le procédé de l'invention est au moins trente deux fois plus sensible que la méthode de Coombs pour le titrage des anticorps anti Rhésus D.

**Revendications**

1. Procédé de détermination d'une cellule ou d'un micro-organisme dans un échantillon,caractérisé par le fait que :

   a) on immobilise, sur une phase solide, un ligand ayant une affinité de fixation pour ladite cellule, ou ledit micro-organisme;

   b)on met à incuber l'échantillon en présence de ladite phase solide sur laquelle est immobilisé le ligand;

   c)on lave la phase solide après incubation;

   d)on révèle la présence de ladite cellule ou ledit microorganisme fixé sur la phase solide par l'intermédiaire du ligand, par mise en évidence d'une activité enzymatique ou chimique endogène de ladite cellule ou dudit micro-organisme après lyse.

2. Procédé selon la revendication 1, caractérisé par le fait que la cellule est un globule rouge, un leucocyte, une plaquette sanguine, une cellule d'origine épithéliale, endothéliale ou conjonctive normale ou pathologique et le micro-organisme est une bactérie, un parasite ou un virus.

3. Procédé selon l'une des revendications 1 ou 2, caractérisé par le fait que le ligand est choisi parmi les lectines, les antigènes et les anticorps.

4. Procédé de détermination d'un antigène de surface d'une cellule, caractérisé par le fait que :

   a) on immobilise sur une phase solide un anticorps correspondant à l'antigène en cause;

   b)on met à incuber l'échantillon contenant ladite cellule en présence de ladite phase solide sur laquelle est immobilisé l'anticorps;

   c)on lave la phase solide après incubation;

   et

   d) on révèle la présence de ladite cellule à l'aide d'une propriété inhérente à celle-ci , notamment par mise en évidence d'une activité enzymatique contenue dans ladite cellule, après lyse de celle-ci.

5. Procédé de détermination,dans un échantillon, du groupe sanguin correspondant aux antigènes de surface des globules rouges, caractérisé par le fait que :

   a)on immobilise sur une phase solide un anticorps spécifique du groupe à déterminer,

   b)on met à incuber l'échantillon en présence de ladite phase solide sur laquelle est immobilisé ledit anticorps;

   c)on lave la phase solide après incubation;

   et

   d) on révèle la présence des globules rouges fixés par l'intermédiaire de l'anticorps précité à l'aide d'une propriété inhérente auxdits globules rouges.

6. Procédé selon la revendication 5, caractérisé par le fait que l'échantillon à déterminer est du sang total ou une dilution de sang.

**7.** Procédé selon l'une des revendications 5 ou 6, caractérisé par le fait que l'anticorps est un anticorps monoclonal.

**8.** Procédé selon l'une des revendications 5 à 7, caractérisé par le fait que les globules rouges sont mis en évidence par leur coloration propre.

**9.** Procédé selon l'une des revendications 5 à 7, caractérisé par le fait que les globules rouges sont révélés par mise an évidence d'une activité enzymatique ou chimique endogène, après lyse.

**10.** Procédé selon la revendication 9, caractérisé par le fait que l'activité enzymatique endogène est l'activité peroxydasique de l'hémoglobine mise en évidence par un substrat chromogène.

**11.** Phase solide pour la mise en oeuvre du procédé selon l'une des revendications 1 à 10, caractérisée par le fait qu'elle comporte plusieurs ligands fixés et qu'elle est modifiée pour être conservée sous forme desséchée ou lyophilisée.

**12.** Phase solide selon la revendication 11,caractérisée par le fait qu'elle est sous forme de bandelette ou de plaque de microtitration ou de billes.

**13.** Phase solide selon la revendication 12, caractérisée par le fait que chaque ligand est déposé sur la bandelette ou la plaque selon une configuration particulière .

**14.** Phase solide selon la revendication 13, caractérisée par le fait que la bandelette comporte un anticorps anti-A déposé sous forme d'un A, un anticorps anti-B sous forme d'un B et/ou un anticorps anti-AB sous forme de AB pour le groupage sanguin.

**15.** Phase solide selon la revendication 12, caractérisée par le fait que chaque plage comportant un ligand présente une indication permettant à l'homme ou à la machine d'identifier la nature du résultat, par exemple un code notamment alphanumérique ou un code à barres .

**16.** Coffret pour la détermination du groupe sanguin conformément au procédé tel que défini à la revendication 10, caractérisé par le fait qu'il comporte:
- une phase solide revêtue de différents anticorps dans des plages différentes,chacun étant spécifique d'un groupe sanguin;
- une solution de lavage;
- un substrat chromogène; et
- un agent de lyse.

**Claims**

**1.** Process for determining a cell or a microorganism in a sample, characterized in that :
a) a ligand having a binding affinity for said cell or said microorganism is immobilized onto a solid phase ;
b) the sample is incubated in presence of said solid phase on which the ligand is immobilized;
c) the solid phase is washed after incubation;
d) the presence of said cell or said microorganism bound onto the solid phase through the ligand is showed by revealing an enzymatic or chemical endogenous activity of said cell or said microorganism after lysis.

**2.** Process according to claim 1, characterized by the fact that said cell is a red blood globule, a leucocyte, a blood platelet, a normal or pathologic cell of epithelial, endothelial or connective origin and said microorganism is a bacteria, a parasite or a virus.

**3.** Process according to any of claims 1 or 2, characterized by the fact that said ligand is selected among lectins, antigens and antibodies.

**4.** Process for determining a cell surface antigen, characterized in that :
a) an antibody corresponding to the concerned antigen is immobilized onto a solid phase ;

9

b) the sample containing said cell is incubated in presence of said solid phase on which the antibody is immobilized;

c) the solid phase is washed after incubation; and

d) the presence of said cell is showed through a property inherent to it, including by revealing an enzymatic activity contained in said cell after lysis thereof.

5. Process for determining in a sample the blood group corresponding to the surface antigens of red globules, characterized in that :

a) a specific antibody for the group to be determined is immobilized onto a solid phase ;

b) the sample is incubated in presence of said solid phase on which said antibody is immobilized;

c) the solid phase is washed after incubation; and

d) the presence of said red globules bound is revealed through said antibody by a property inherent to said red globules.

6. Process according to claim 5, characterized by the fact that the sample to be analysed is pure blood or a blood dilution.

7. Process according to any of claims 5 or 6, characterized by the fact that the antibody is a monoclonal antibody.

8. Process according to any of claims 5 to 7, characterized by the fact that the red globules are revealed by their own coloration.

9. Process according to any of claims 5 to 7, characterized by the fact that the red globules are revealed by an endogenous enzymatic or chemical activity after lysis.

10. Process according to claim 9, characterized by the fact that said endogenous enzymatic activity is the peroxydase activity of hemoglobine revealed by a chromogenic substrate.

11. Solid phase for carrying out the process according to any of claims 1 to 10, characterized by the fact that it includes various bound ligands and is modified so as to be stored under a dried or freeze-dried form.

12. Solid phase according to claim 11, characterized by the fact that it is present under the form of a micro-assay striplet or plate or balls.

13. Solid phase according to claim 12, characterized by the fact that each ligand is applied onto the striplet or plate with a special configuration.

14. Solid phase according to claim 13, characterized by the fact that said striplet includes an anti-A antibody applied under an A form, an anti-B antibody applied under an B form and/or an anti-AB antibody applied under an AB form for blood grouping.

15. Solid phase according to claim 12, characterized by the fact that each plaque including a ligand has an indication to allow an operator or a machine to identify the nature of the result, for example, mainly an alphanumeric code or a barcode.

16. Kit for determining the blood group by a process as defined in claim 10, characterized by the fact that it includes :

- a solid phase coated with different antibodies in different plaques, each one being specific for one blood group;
- a washing solution;
- a chromogenic substrate; and
- a lysis agent.

**Patentansprüche**

1. Verfahren zur Bestimmung einer Zelle oder eines Mikroorganismus in einer Probe, dadurch gekennzeichnet, daß:

   a) man auf einer festen Phase einen Liganden immobilisiert, der eine Fixierungsaffinität für die Zelle oder den Mikroorganismus besitzt,

   b) man die Probe in Gegenwart der festen Phase, auf der der Ligand immobilisiert ist, bebrütet,

   c) man die feste Phase nach dem Bebrüten wäscht,

   d) man die Gegenwart der Zelle oder des Mikroorganismus, der auf die feste Phase durch die Zwischenstufe des Liganden fixiert ist, durch das Vorhandensein einer enzymatischen Aktivität oder chemisch-endogenen Aktivität der Zelle oder des Mikroorganismus nach der Lysis aufzeigt.

2. Verfahren nach Anspruch 1, dadurch gekennzeichnet, daß die Zelle ein rotes Blutkörperchen, ein Leukozyt, ein Blutplättchen, eine Zelle epithelialen oder endothelialen Ursprungs ist oder aus der normalen oder pathologischen Bindehaut stammt und dass der Mikroorganismus eine Bakterie, ein Parasit oder ein Virus ist.

3. Verfahren nach einem der Ansprüche 1 oder 2, dadurch gekennzeichnet, daß der Ligand ausgewählt ist aus Lectinen, Antigenen und Antikörpern.

4. Verfahren zur Bestimmung eines Oberflächenantigens einer Zelle, dadurch gekennzeichnet, daß:

   a) man auf einer festen Phase einen dem betreffenden Antigen entsprechenden Antikörper immobilisiert,

   b) man die Probe, die die Zelle enthält, in Gegenwart der festen Phase, auf der der Antikörper immobilisiert ist, bebrütet,

   c) man die feste Phase nach der Bebrütung wäscht,

   d) man die Gegenwart der Zelle mit Hilfe einer ihr innewohnenden Eigenschaft aufzeigt, insbesondere durch das Aufzeigen einer enzymatischen Aktivität der Zelle nach ihrer Lysis.

5. Verfahren zur Bestimmung der Blutgruppe in einer Zelle, die sich auf die Oberflächenantigene der roten Blutkörperchen bezieht, dadurch gekennzeichnet, daß:

   a) man auf einer festen Phase einen spezifischen Antikörper der zu bestimmenden Gruppe immobilisiert,

   b) man die Probe in Gegenwart der festen Phase, auf der der Antikörper immobilisiert ist, bebrütet,

   c) man die feste Phase nach dem Bebrüten wäscht,

   d) man die Anwesenheit der roten Blutkörperchen, die durch das niedergeschlagene Zwischenprodukt des Antigens fixiert sind, mit Hilfe einer den roten Blutkörperchen innewohnenden Eigenschaft aufzeigt.

6. Verfahren nach Anspruch 5, dadurch gekennnzeichnet, daß die zu bestimmende Probe Gesamtblut oder verdünntes Blut darstellt.

7. Verfahren nach einem der Ansprüche 5 oder 6, dadurch gekennzeichnet, daß der Antikörper ein monoclonaler Antikörper ist.

8. Verfahren nach einem der Ansprüche 5 bis 7, dadurch gekennzeichnet, daß die roten Blutkörperchen durch ihre eigene Farbe angezeigt werden.

9. Verfahren nach einem der Ansprüche 5 bis 7, dadurch gekennzeichnet, daß die roten Blutkörperchen durch das Vorhandensein einer enzymatischen Aktivität oder einer chemisch-endogenen Aktivität nach der Lysis angezeigt werden.

10. Verfahren nach Anspruch 9, dadurch gekennzeichnet, daß die enzymatisch-endogene Aktivität die Peroxydase-Aktivität des Hämoglobins ist, die durch ein chromogenes Substrat aufgezeigt wird.

11. Feste Phase zur Durchführung des Verfahrens nach einem der Ansprüche 1 bis 10, dadurch gekennzeichnet, daß sie mehrere fixierte Liganden enthält und daß sie modifiziert ist, damit sie in getrockneter oder lyophilisierter Form aufbewahrt werden kann.

**12.** Feste Phase zur Durchführung des Verfahrens nach Anspruch 11, dadurch gekennzeichnet, daß sie in Form eines Mikrotritierstreifens oder -plättchens oder von Kügelchen vorliegt.

**13.** Feste Phase nach Anspruch 12, dadurch gekennzeichnet, daß jeder Ligand auf dem Streifen oder Plättchen gemäß einer bestimmten Konfiguration abgeschieden wird.

**14.** Feste Phase nach Anspruch 13, dadurch gekennzeichnet, daß der Streifen einen Anti-A-Antikörper enthält, der in Form eines A niedergeschlagen wird, einen Anti-B-Antikörper, der in Form eines B und/oder einen Anti-AB-Antikörper, der in Form eines AB für die Blutgruppe abgeschieden wird.

**15.** Feste Phase nach Anspruch 12, dadurch gekennzeichnet, daß jeder einen Liganden enthaltende Bereich einen Hinweis ergibt, gemäß dem es dem Menschen oder der Apparatur möglich ist, die Art des Resultats zu erkennen, z. B. insbesondere einen alpha-numerischen Code oder einen Strichcode.

**16.** Kit zur Bestimmung der Blutgruppe gemäß dem Verfahren nach Anspruch 10, dadurch gekennzeichnet, daß es enthält:
- eine feste Phase, ausgestattet mit verschiedenen Antikörpern für verschiedene Bereiche, wobei jeder spezifisch für eine Blutgruppe ist;
- eine Waschlösung
- ein chromogenes Substrat
- ein Mittel für die Lysis

Sang
de
groupe A

Sang
de
groupe B

Sang
de
groupe O

Figure 1

DEPISTAGE DES ANTICORPS ANTI ERYTHRANTIGENE
A OU B SELON LA METHODE 1

Figure 2

DEPISTAGE DES ANTICORPS ANTI RHESUS D
SELON LA METHODE II

Figure 3

EP 0 217 845 B1